# EUROPEAN PATENT SPECIFICATION

(11) **EP 0 355 140 B1**
(45) Date of publication and mention of the grant of the patent: **20.03.1996**
(21) Application number: 89900809.8
(22) Date of filing: 14.11.1988
(51) Int. Cl.: C12P 21/00, A61K 39/42, G01N 33/569

(54) **HUMAN MONOCLONAL ANTI-HIV-I-ANTIBODIES**
MONOKLONALE MENSCHLICHE ANTIKÖRPER GEGEN HIV-I
ANTICORPS MONOCLONAUX HUMAINS ANTI-HIV 1

(30) Priority: 13.11.1987 US 120489
(43) Date of publication of application: 28.02.1990
(73) Proprietor: KATINGER, Hermann, A-1190 Wien (AT)
(72) Inventor: KATINGER, Hermann, A-1190 Vienna (AT); VON BAEHR, Rüdiger, DDR-1020 Berlin (DD); JUNGBAUER, Alois, A-1210 Vienna (AT); PORSTMANN, Tomas, DDR-1055 Berlin (DD); STEINDL, Franz, J., A-1160 Vienna (AT); GRUNOW, Roland, DDR-1055 Berlin (DD); BUCHACHER, Andrea, A-1190 Vienna (AT)
(74) Representative: Büchel, Kurt F., Dr.
(86) International application number: PCT/EP88/01072
(87) International publication number: WO 89/04370

(56) References cited:
- EP-A- 0 251 612
- Interferon Y Biotecnologia, vol. 4, no. 3, 1987; R.Grunow et al.: "Obtencion de un heteromieloma de cultivo (CB-F7) con alta eficiencia para la inmortalizacion de cèlulas B.", pages 261-264
- Journal of Immunological Methods, vol. 106, 10 February 1988, Elsevier Science Publishers B.V., NL; R. Grunow et al.: "The high efficiency, human B cell immortalizing heteromyeloma CB-F7", pages 257-265
- Biochemical and Biophysical Research Communications, vol. 155, no. 3, 30 September 1988, Academic Press, US; T. Sugano et al.: "Human monoclonal antibody against glycoproteins of human immunodeficiency virus", pages 1105-1112

## Description

### FIELD OF THE INVENTION

This invention is in the field of immunology specially AIDS therapy and in vivo imaging of HIV-Infected cells. More particulary it concerns a human monoclonal anti-HIV-1-antibody and immunochemicals made from this antibody and therapeutic methods that use these immunochemicals. The production of human monoclonal antibodies (hu mAbs) may present a new source of antibodies to be used in immunotherapy for infections and other diseases. The application of mAb of human origin instead of murine origin avoids the induction of antibody response in humans. The Fc-part of murine antibodies has to be removed prior to injetion in humans to minimize antibody response against heterogenous administered proteins. Human monoclonal antibodies however present a part of the homologous proteins of human individuals. In the sera of human immunodeficincy virus (HIV) infected men anti-virus antibodies could be detected over a certain period after infection without any clinical manifestations of the aquired immunodeficiency syndrome (AIDS). At this state of active immune response high numbers of antigen-specific B cells were expected in the circulation. These B-cells were used as fusion partners for the generation of human monoclonal anti HIV antibodies. It is described herein:
- the generation of human mAb to HIV antigenes
- the substantial inhibition of growth of HIV-Infected cells
- the preparation of immunotoxins conjugated with the described human anti-HIV-1-mAb and an A-chain toxin
- the selective killing of HIV-1 infected cells with the immunotoxin
- the selective killing of HIV-1 infected cells by antibody dependent cellular cytotoxicity
- the selective prevention of infection of cells with the human monoclonal anti-HIV-1 antibody
- The selective determination of HIV-1 antigen in human sera

### STATE OF THE ART

EP-A-0 251 612 discloses an antibody which binds selcetively to an epitope of a protein of HIV-I. This antibody showed different binding specifity to the antibody of the invention.

### BRIEF DESCRIPTION OF THE INVENTION

The invention consists in the antibody 3D6 which binds selectively to an envelope protein of HIV-I produced by hybridoma with the ECACC accession numbers 87110301.

### DETAILED DESCRIPTION OF THE INVENTION

### Materials and Methods Human anti-HIV-monoclonal antibodies (HU anti-HIV-1-mAb)

In the presently preferred ambodiment of the invention the hu anti-HIV-1-mAb used is of IgG₁ subclass produced according to known hybridization procedures described by R. Kennet et al. in "Monoclonal antibodies and functional cell lines; progress and applications". Plenum Press (New York), 1984. Samples of hybridoma 3D6 were deposited in ECACC, Porton Down, Salisbury SP4 OJG, UK.The ECACC accession number and deposit date for the deposited hybridoma is: 87110301; November 3^{rd} 1987.

### Fusion

Fusion is performed in a described manner (Köhler et al., Europ. Journal of Immunology, 6 (1976):292). Prior to cell fusion with PEG 1500 peripheral blood lymphocytes from HIV-I serum positive donors were washed 3 times with serum free cell cultur medium. The cells were fused immediately or stimulated with 5 ng pokeweed mitogen (PWM) for three days prior to fusion. The cells were mixed at a ratio of 5:1 with HAT-sensitive fusion cells. The cells were fused with 42 % PEG 1500 (Ferak,FRG) in the presence of 7.5 % DMSO (Serva,FRG). Cells were cloned and subcloned by limiting dilution.

### Screening of the Hybridomas

IgG and IgM isotypes were analyzed from the culture supernatant by a sandwich enzyme linked immunosorbant assay (ELISA) with peroxidase conjugated anti human IgG, IgM (heavy chain specific) antibodies in a known manner. Screening for specific antibodies was performed by a sandwich ELISA using HIV in a concentration of 10 microgram/ml. The virus isolated from tissue culture was coated in flat bottom microtiterplates and the unbound material was washed out. The culture supernatants were incubated overnight at 4°C. After washing out unbound material the specific antibodies were detected by anti human-Ig-heavy-chain specific antibodies labelled with peroxidase.

### Immunoblotting

Immunoblot using HIV envelope proteins prepared from native virons: Purified virus was denaturated and reduced with 2.5 % SDS and 5.0 % 2-mercaptoethanol at 90° C for 5 min and applied to a 10 % polyacrylamide slab gel. After separation protein bands were electro-transferred into a nitrocellulose sheet (Schleicher & Schüll, FRG). After blocking the sheet with 5 % dry milk, strips were cut and immersed in 5 ml 1:2 diluted culture supernatant. Bound antibodies were detected by reacting with anti-human IgG antibodies labelled with HRP and staining with diaminobenzidine containing 0.1 % NiCl₂.

### Immunoblot using HIV envelope proteins prepared from cloned material

SDS-gelelectrophoresis was performed according to Laemmli, (Nature 227, 1970; 680-685). The electrophoretically separated envelope or core proteins resp.derived from genetically engineered E. coli (L.H. Gosting, et al., Journal of Clinical Microbiology 52 1987 (845-848)) were electroblotted to nitrocellulose after blocking with 3 % bovine serum albumin (BSA dissolved in PBS) the strips were immersed in samples diluted in PBS buffer (containing 0.1 % Triton X-100, 1 % BSA, 0.5 % gelatine) overnight,washed 3 times and incubated with goat anti human GAMMA-chain labelled with peroxidase.

### Immunoblots using commercially available blot strips:

Blot strips from Du Pont Lot 7044128 were used according to recommendations of the company

### Commercial available EIA

Commercial available EIA were used to check the specifity of monoclonal antibodies. Both, catch-elisa and competitive EIA were used. Assays from Labsystems (Finland), Organon (Belgium), Pasteur (France), Biochrom (FRG), Abott (USA) and a competitive EIA from Organon were used.

### Peptide mapping

Different peptides were used for the evaluation of the antigen binding sites.The peptides A and B were synthesized according to literature. C and D were a gift from Biochrom (FRG). 4 differnt peptides were tested.
Peptid A: Wang et al. PNAS 83 6159-6163 (1986
RILAVERYLKDQLLGIWGCS
Peptide B: Gnann et al. J. Virol.61 2639-2641 (1987)
LGIWGCSGKLIC
Peptide C: own results
KDQQLLGIWGCSGKLIC
Peptide D: obtained from Biochrom
KDQQLLGIWGCSGKLICVPWNAS

The peptides C and D reacted with the monoclonal antibody produced by the hybridoma no. 3D6.

### Hybridization detection of human DNA

10⁶ cells of each cell line were spotted on a nitrocellulose membrane. The hybridization probe was the ³P-labelled plasmid Blur8 which contains a cloned Alu-sequence that is specific for human DNA and repeated about 300.000 times in the genome (Schmid et al., Science 216, 1982; 1065). After prehybridization and hybridization overnight the membrane was washed 3 x in 100 ml 2 x SSC, 0.1 % SDS for 5' at room temperature and 2 x in 200 ml 1 x SSC, 0.1 % SDS for 30' at 60°C and autoradiographed for 80 hours.

### Immunofluorescence

Fixed HIV-1 infected cells (H9 cells) were incubated with the hu anti-HIV-mAb. Binding of the mAb was demonstrated by a second incubation after washing out unbound mAb with FITC labelled anti human IgG.

### Purification of the 3D6 antibody

Sephadex, Sepharose and Sephacyl are trade marks. Purification was performed in a known manner. The clarified culture supernatant was desalted using gelfiltration on sephadex G-25 column (Pharmacia) and further chromatographed on a CM-Sepharose fast flow column (Pharmacia). The eluate of the CM-Sepharose fast flow column was concentrated by ultrafiltration and rechromatographed using Phenyl-Superose (Pharmacia). The chromatographic steps were performed according to the recommendations of the Pharmacia company. Prior to the loading of the solution on the Phenyl-superose column the crude mAb solution was diluted by a 2 M-ammonium sulfate solution.

### Preparation of Ricin-A-chain Toxin

Ricin is extracted from castor beans (Ricinus Communis) by known methods. Ricin is extracted either from grinded whole castor beans (Ricinus Communis) or from castor bean cake, which is a byproduct of castor bean processing. The castor bean cake is defatted by extraction 3 times with 5 volumes of 40 % - 60 % (v/w) petroleum ether. The air-dried material is then extracted over night in phosphate buffered saline (PBS; 0.15 M NaCl, 0.01 M phosphate, pH 7.2).The extract is cleared by filtration through a nylon gauze followed by centrifugation at 1500 g for 1 hour. The clear super natant is precipitated at 4° C with saturated ammonium sulfate. At a final concentration of 60 % ammonium sulfate the precipitate is collected and harvested by centrifugation (1500 g, 1 hour) redissolved in a minimum amount of PBS and dialyzed against PBS until the extract is ammonium sulfate free.

### Affinity chromatography

The clarified and dialyzed toxin extract is further purified by lectin affinity chromatography. The gel (Sepharose 4B, Fa. Pharmacia) is pretreated with 1 M propionic acid at room temperature for at least 4 weeks to enhance its binding capacity for lectins.

The column chromatography should be operated at temperatures lower than 10° C to optimize lectin binding.

The clarified and dialyzed extract is applied to a PBS equilibrated acid pretreated Sepharose 4B-column. After sample application the column is washed with PBS until the UV-absorbance return to the baseline. The toxin is eluted together with other lectins with 100 mM galactose in PBS. This mixture is further separated by gelchromatography on Sephacryl S200 HR. The sample volume should not exceed 3-4 % of the bed total volume. The toxin is resolved completely from the other lectins under these conditions. The toxin recovered from the affinity column is concentrated to 10 mg/ml by ultrafiltration (Milipore PTGC membrane) prior to application on the Sephacryl S200 HR column. The toxin peak was collected and filter sterilized. The sterile toxin solution was stored deep frozen at -30°C until cleavage into A and B-chain.

### Cleaveage of Ricin into A- and B-chain

The affinity purified and concentrated toxin is cleaved into A- and B-chain by a 5 % beta-mercaptoethanol solution. The toxin is incubated at a concentration of 5 mg/ml in 0.1 M Tris-HCl pH 8.5 buffer completed with beta-mercaptoethanol (5 % end concentration) and galactose (0.5 M final concentration) overnight at room temperature, followed by 2-3 hours at 37° C. The toxin is transferred from the starting buffer (PBS) into the incubation buffer (Tris buffer) by gelchromatography on Sephadex G25 columns equilibrated with the incubation buffer.

After incubation the sample is applied to a DEAE-Sepharose fast flow equilibrated with 0.1 M Tris/HCl buffer pH 8.5. The column is washed with 0.1 M Tris/HCl buffer pH 8.5 until all unbound material is eluted. The unbound material, essentially pure A-chain, is collected and passed down an asialo-fetuin-Sepharose 4B column to remove contaminating toxin. The asialo fetuin-Sepharose 4B was prepared according to the recommendation of Pharmacia company. The unbound material is collected and filter sterilized and stored at 4° C.

The DEAE-Sepharose fast flow is regenerated with a 0.1 M Tris-HCl buffer pH 8.5 containing 0.1 M Galactose and 1 M NaCl.

### Determination of the isoelectric point

The isoelectric point of the hu anti-HIV-1-mAb was determined by a described method. Pharmalytes were used as carrier ampholytes. The whole procedure was carried out according the recommendation of the Pharmacia company (booklet: Isoelectric focusing, Pharmacia fine chemicals).

### Determination of subclass and light chains

Light chains and subclass were determined by ELISA. Specific anti human KAPPA-chain antibodies labelled with alkaline phosphatase or specific anti human G1, G2, G3 and G4 antibodies labelled with Peroxidase were used.

### Quality control of Ricin A-chain

Quality control tests are performed on the purified Ricin-A chain. Gradient SDS-polyacrylamide gel electrophoresis under reduced conditions shows the absence of any contaminating material. Only one band at 33 and one at 30 kilo dalton respectively can be detected.

### Conjugation of A-chain toxin with monoclonal antibody

5 mg of purified monoclonal antibody (1-2 mg/ml in PBS) were reacted with a 10-fold molar excess of SPDP (Pharmacia), dissolved at 1 mg/ml in dimethylformamide for 30 min at room temperature. PDP-substituted antibody was desalted by gelfiltration using Sephadex G-25. The protein peak as determined continuously at 280 nm was collected and placed on ice. Five mg of ricin A chain were reduced with 5 mM DTT for 1 hour at room temperature and desalted on a column of Sephadex G-25. The column was equilibrated with PBS. The protein peak was collected and was immediately mixed with the cold PDP-substituted antibody. The mixture was rocked for 1 hour at 4°C and then diafiltrated at 4°C against 0.01 M sodium phosphate buffer containing 2.0 M sodium chloride. The bulk of the unconjugated A-chain was separated from the immunotoxin by gelchromatography on Sephacryl S200 HR. The column was equilibrated in 0.02 M sodium phosphate containing 3 M sodium chloride.

The first peak, which contained the immunotoxin was pooled and affinity purified on an anti-human IgG-Sepharose 4B. The immunotoxin was eluted from the affinity column with 3.5 M magnesium chloride and extensively dialyzed against sodium chloride and then against PBS.

Immunotoxins (0.2-2 mg/ml) were concentrated by ultrafiltration using PTGC membranes to the end concentration necessary for testing. The final preparation was sterile filtered and stored in aliquots at -20°C. The immunotoxins were analyzed by SDS-PAGE under both reducing and nonreducing conditions. The substitution of the immunotoxin was determined by radioimmunoassay. An average substitution of 1-2 moles A-chain per mole antibody was observed.

### Competitive EIA

Purified hu anti-HIV-mAb were conjugated with Peroxidase by a known method according to M.B. Wilson and P.K. Nakane (1978) in "Immunoflourescence and related techniques", ed. W. Knapp et al. Elsevier Netherlands.

A dilution series of hu anti-HIV-mAb (in PBS 1 % BSA and 0.1 % Triton X-100) was dispensed in flat bottomed microtiter wells coated with purified HIV envelope proteins incubated overnight at 4°C. After washing out unbound material the bound conjugate was determined by reaction with 1.4 phenylendiamine. The developed colour was measured at 492 nm. A calibration curve was blotted optical density versus dilution. The dilution of the half saturation is calculated from this calibration curve.

Hu anti-HIV-mAb at a dilution corresponding to the half maximum saturation was mixed with sera from patients suffering from AIDS or with sera from probands who were seropositiv determined by a conventional screening (ELISA) followed by a confirmation test (westernblot). The mixture of the conjugate (at half maximal dilution) and the serum from the probands were incubated in HIV envelope protein coated microtiter wells, washed and stained.

The optical density of these samples is compared to the anti HIV mAb at half maximum saturation. A value below the half maximum saturation indicates a competition between the human sera and the human monoclonal anti-HIV antibody.

### Neutralizing activity on the infectivity of HIV-1 virions

The assay for neutralizing activity of the hu anti-HIV-1-mAb was performed according to a known method (J. Virology, 61,2024-2028, 1987), Nature 316, 72-74, 1985; Biotechnology 5, 940-946, 1987). An HIV-1 dose equivalent to twenty times the amount to cause infection in 50 % inoculated cultures on day 12 (20 x TICD 20) was used to infect the H9-cell line. Cells were collected 3,6,9 and 12 days after virus inoculation and percentage of infection was determined by immunofluorescence. 100 % infection was observed between day 6 and 9. The neutralizing effect was also tested in the presence of the monoclonal antibody at variable dilutions.

The neutralizing activity of the 3D6 antibody was evaluated by addition of a single dose of antibody (final concentration 1µg/ml) immediately after virus inoculation.

The infection rate was expressed as percent of infected cells.

### Cytotoxicity tests

The test cell line used in the cytotoxicity tests were HIV-1 infected H9 cells . HIV-1 infected H9 cells were prepared as described under Neutralizing activity on the infectivity of HIV-1 virions. Uninfected H9 cells were used as negative control. Cells harvested after a period of 6-9 days after virus inoculation were suspended in 1 ml medium. Various dilutions of the hu mAb-toxin conjugate were added to the infected cells and the negative control. After incubation for 24 hours at 37°C cells were washed with PBS and methionine free medium supplemented with ³⁵S-methionine was added. Cells were incubated for 2 hours at 37°C, then the medium was removed by centrifugation and the cells transferred to a glass fiber filter and washed three times with 10 % trichloracetic acid, containing 1 mg/ml methionine. The cells were air dried on the filter and then transferred into a scintillation fluid. Radioactivity was counted in a scintillation counter. Cytotoxicity was expressed as the tissue culture inhibitory dose of the conjugate that resulted in 50 % of control untreated protein synthesis (TCID₅₀).

### EXPERIMENTAL RESULTS

A variety of experiments are shown to serve as examples for illustrating the present invention and its utilization in the following embodiments.

The human origin, the biochemical properties, the immunochemical characterization, the in vitro and in vivo behaviour are demonstrated by representative results.

In the drawings show:
Fig. 1a: Immunoblot of the human mAb produced by Hybridoma No 3D6. HIV envelope proteins were prepared from native virons by SDS and 2-mercaptoethanol treatment.

Fig. 1b: Immunoblot of the human anti HIV-mAb produced by Hybridoma 3D6 HIV envelope proteins were prepared from genetically engineered E. coli (according to a known procedure, L.H. Gosting et al.: Journal of Clinical Microbiology 25,1987 (845-848).

Fig. 1c: Immunoblot o the human anti HIV mAb produced by Hybridoma 3D6. A commercially available blot strip (Du Pont Lot.: 70 44 128) was used.

Fig. 2: Immunoflourescence of fixed HIV-1 infected H9-cells imaged with a sandwich of 3D6-antibody and anti human IgG conjugated with FITC. The clone 3D6 is shown.

Fig. 3: Neutralizing activity of 3D6-antibody expressed as percent of infected cells. Infected cells were shown by immun flourescence using 3D6 antibody.

Fig. 4: Inhibition of proteinsynthesis measured by ³⁵S-methionine uptake was taken as measure for cytotoxicity on the basis of assuming a molecular weight of 180000 dalton the molar concentration of the immunotoxin was calculated. The TCID₅₀ of the immunotoxin (3D6)-Ricin-A-chain) is less than 10 nm.

Fig. 5: Comparison between 3D6 antibody and sera from seropositives and patients suffering from AIDS or pre AIDS. The results according table II are shown in this figure.

Fig. 6: Commercial available EIA

### Generation of clones and human origin of the monoclonal antibodies

A variety of clones was obtained by the fusion of peripheral blood lymphocytes and the fusion cell line. The specificity of 11 hybridomas is shown in table I.

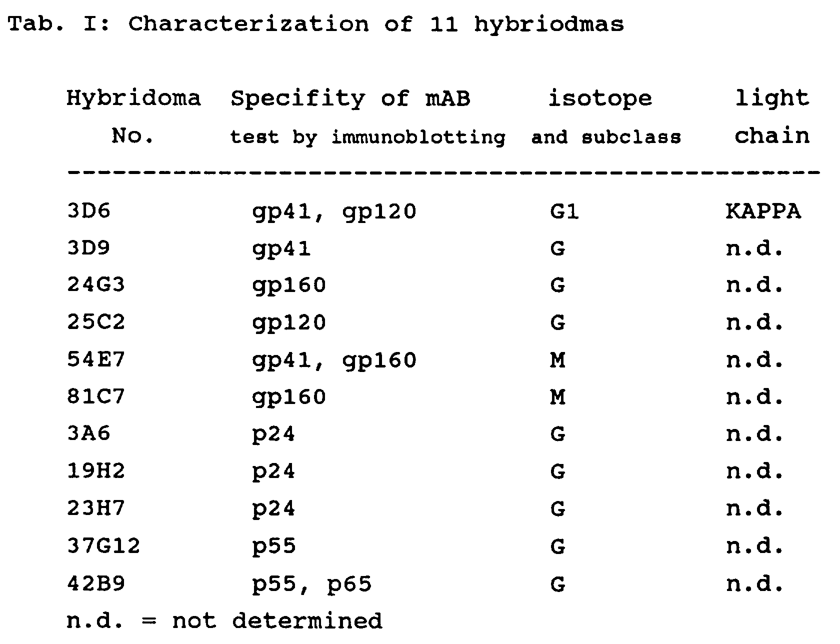

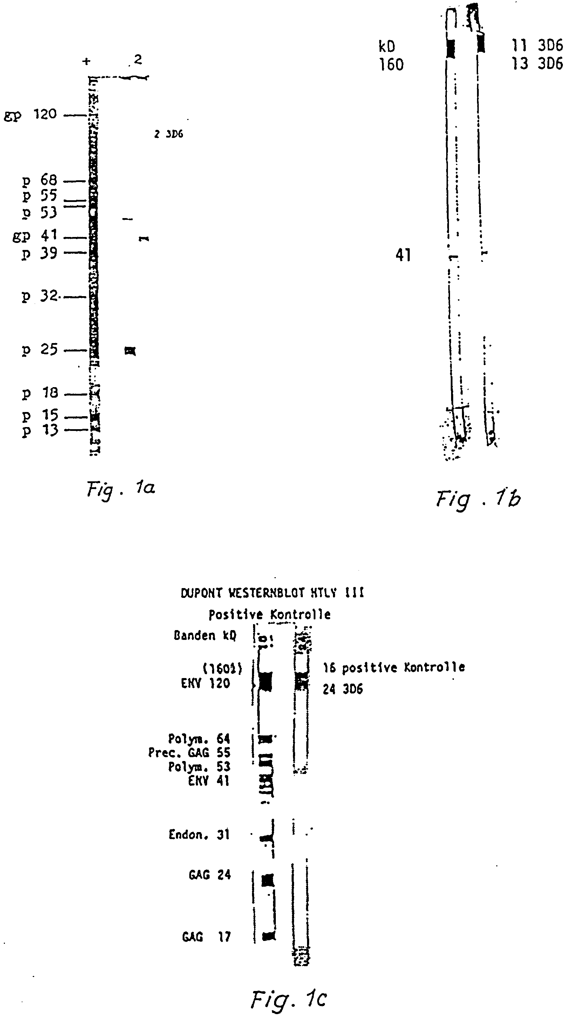

The hybridoma No. 3D6 is deposited in the ECACC, Porton Down, Salisbury, UK under the deposition NO. 87110301 (3^{rd} November 1987). The hu mAb anti HIV-1 produced by this hybridoma line (3D6) is used as example to illustrate the present invention. Immunoblots of the 3D6 antibody are shown in Fig. 1a, b and c.

### Biochemical properties and immunochemical characterization of the antibody

The isotype of the monoclonal antibody produced by Hybridoma No. 3D6 is G. The subclass determined by ELISA is G1. The isoelectric point, determined by isoelectric focusin using the Pharmacia the immobiline system is in the range of pH 8.6.

A summary of the results is also shown in table I

### Immunoflourescence

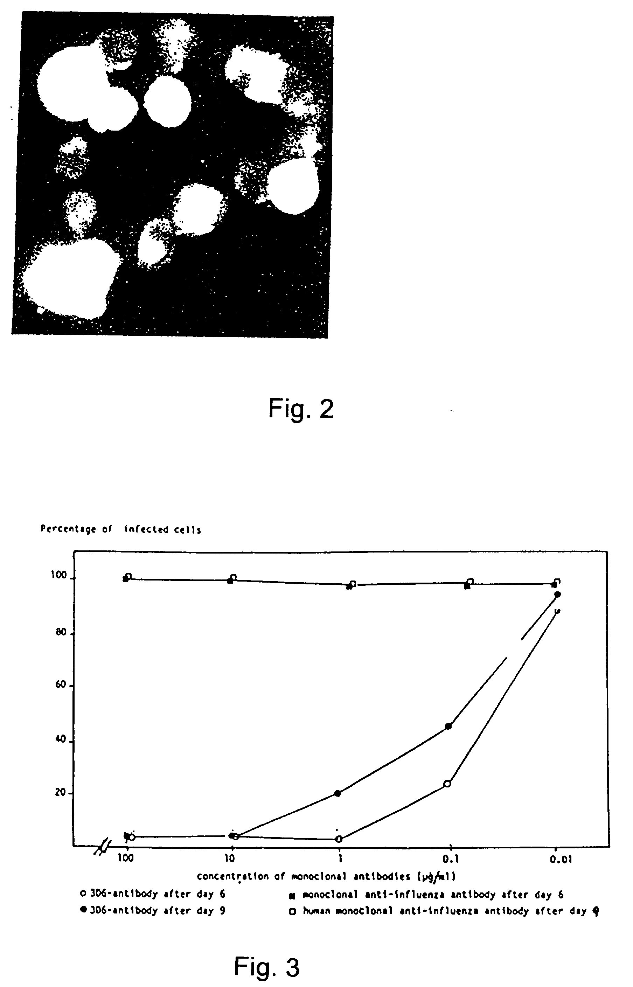

Fixed HIV infected cells were incubated with the 3D6 antibody. Specific flourescence caused by the marker conjugate anti human IgG-FITC is shown in Fig. 2.

### In vitro behaviour of the monoclonal antibodies and related immunotoxins

### Neutralizing activity

In the presence of up to 1 µg/ml 3D6 antibody H9 cells could not be infected by 20 times TCID₅₀ of HIV virons. The neutralizing activity is shown in Fig. 3.

9 days after virus inoculation with 20 time TCID₅₀ 0.1 µg/ml 3D6 antibody could prevent HIV infection in H9 cells.

### Cytotoxicity

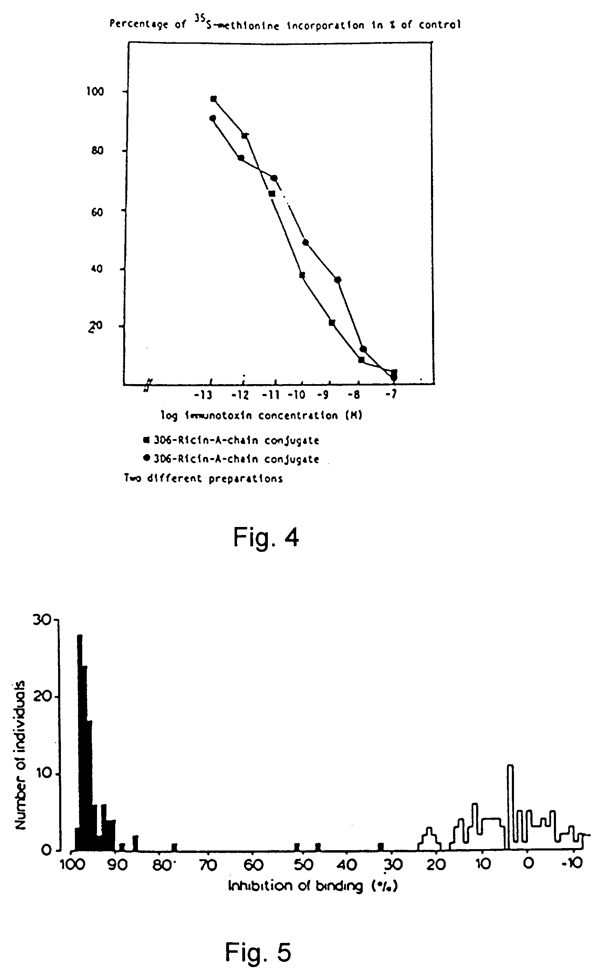

The monoclonal antibody (3D6) covalently linked with the Ricin-A-chain was used to demonstrate the cytotoxicity of the antibody conjugate. The cytotoxicity was measured by ³⁵S-methionine uptake. The immunotoxin consisting of a conjugate between 3D6 antibody and Ricin A-chain, prepared as described under materials and methods killed specifically HIV-1 infected H9 cells. The ³⁵S-methionine uptake is expressed in percent of control (Fig. 4).

### Comparison of the hu anti-HIV-mAb with naturally occuring antibodies from seropositives and patients suffering from AIDS or pre AIDS

For the comparison of the claimed monoclonal antibodies with naturally occuring antibodies in men after a HIV infection a competitve EIA (Enzyme immunoassay) was choosen. The 3D6 monoclonal antibody was conjugated with peroxidase and the dilution of the half maximal saturation was determined. The 3D6 antibody at a dilution of half maximal saturation was mixed with different sera from seropositives without clinical manifestation of AIDS and with sera from patients suffering from AIDS or pre AIDS in a ration of 1:1. The competition between the 3D6 antibody and the natural occuring antibodies in blood are expressed in % of half maximal saturation. The half maximal saturation by the 3D6 antibody is taken as 100 %. Table II shows the values from competitive EIA using a 3D6-antibody peroxidase conjugate and different sera. Fig. 5 shows the results graphically processed.

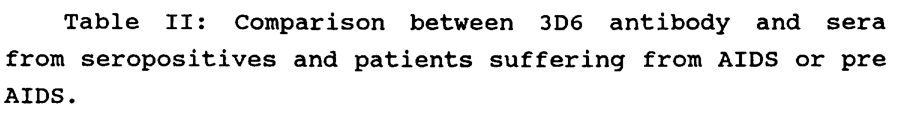

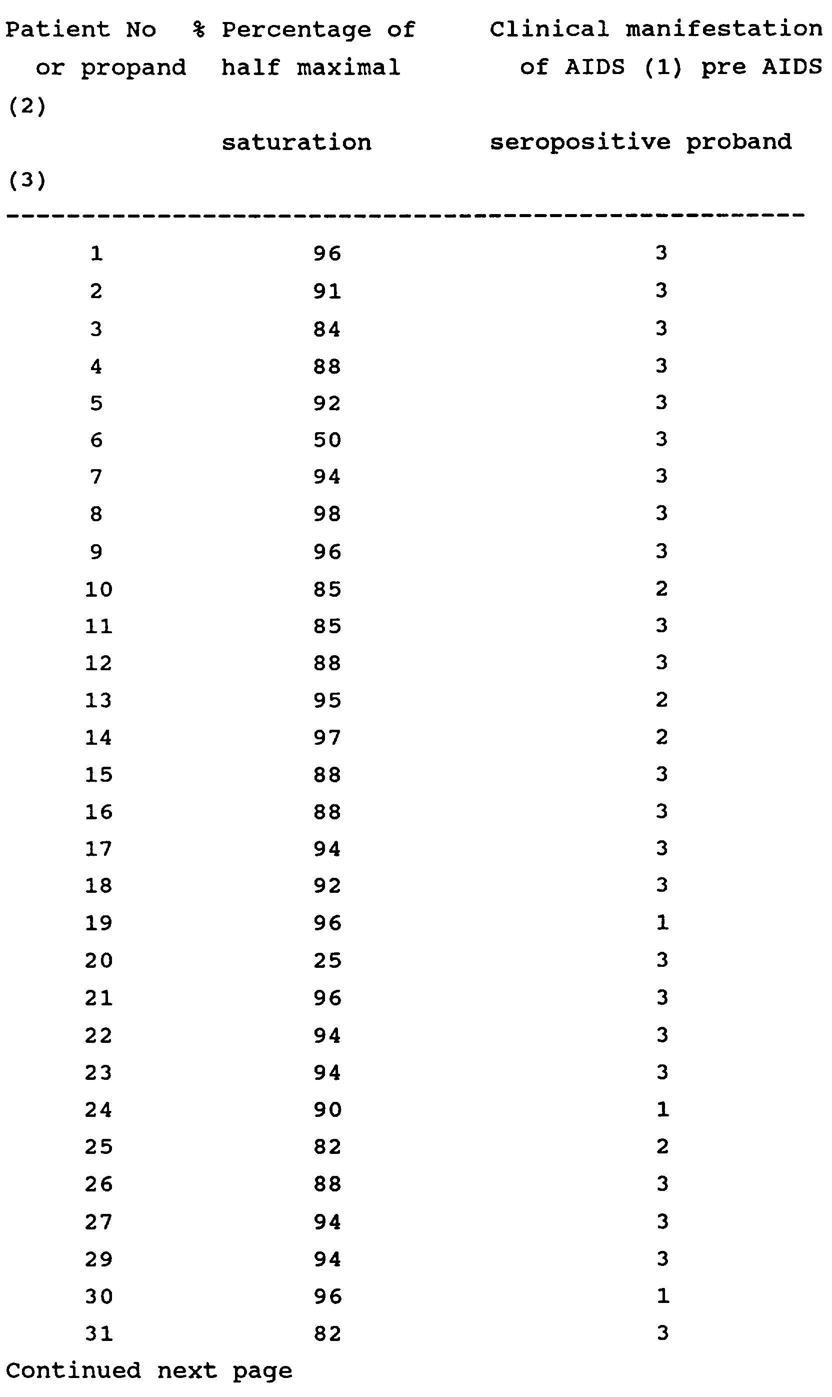

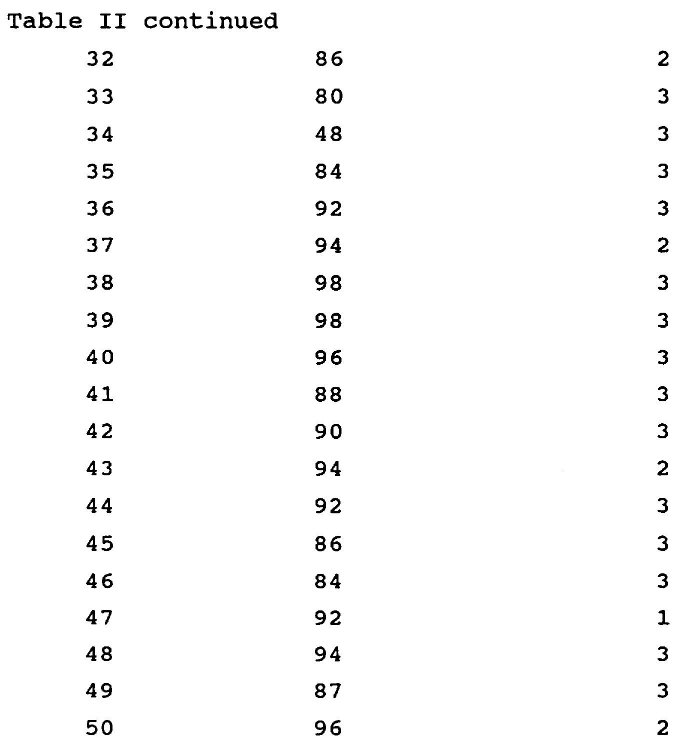

### Peptide analysis

Peptide C and D reacted with the 3D6 antibody. A similar repetitive sequence is also found in gp 120. This is the reason for reaction with gp 41 and gp 120.

### Commercially available EIA

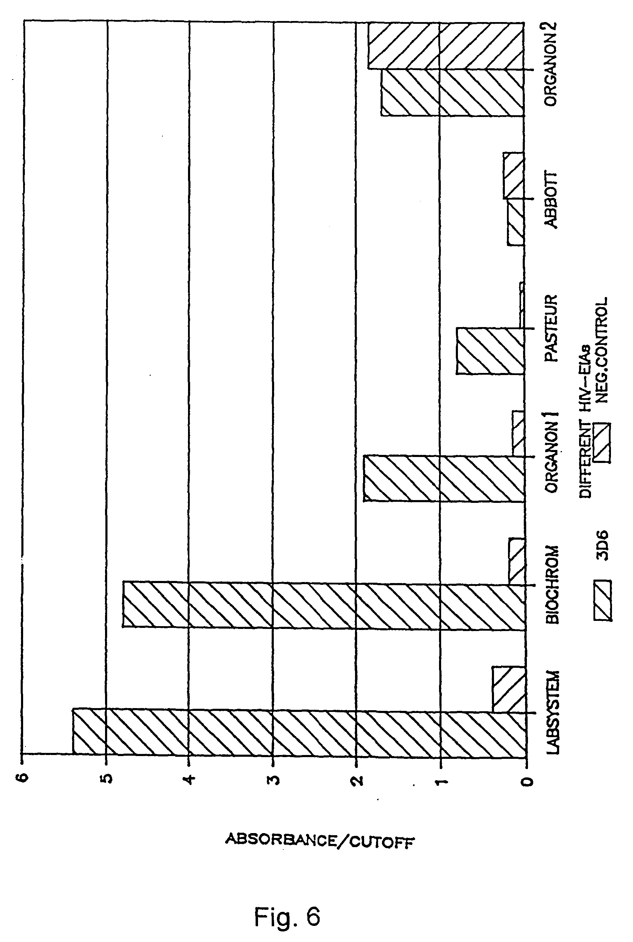

The results of commercially available EIA are shown in Fig. 6. In all EIA a positive result was obtained, except using the Abott ELISA. The Organon (2) assay is a competitive EIA.

## Claims

1. Antibody 3D6 which binds selectively to an envelope protein of HIV-I produced by hybridoma with the ECACC accession numbers 87110301.

2. Antibodies according to claim 1, which also bind selectively to HIV-I (HTLV III/LAV) infected cells.

3. Antibody according to claim 1 which exhibits a TCID 50% against HIV-I infected H-9 cells of less than 10 nM, when conjugated to ricin A-chain.

4. Antibody according to claim 1 or 2 which have a G isotype.

5. A mouse x human hybridoma, which produces a human monoclonal antibody according to one of the claims 1 to 4 and progeny of said hybridomas.

6. An immunotoxin, comprising a conjugate of the antibody according to one of the claims 1 to 4 and a cytotoxic moiety.

7. An immunotoxin according to claim 6, wherein the cytotoxic moiety of said immunotoxin is the A-chain of ricin.

8. An immunotoxin according to claim 6, wherein the cytotoxic moiety of said immunotoxin is the A-chain of abrin.

9. An immunotoxin according to claim 6, wherein the cytotoxic moiety of said immunotoxin is the A-chain of the diphteria toxin.

10. An immunotoxin, comprising a conjugate of the antibody according to one of the claims 1 to 4 and a cytotoxic moiety.

11. An immunotoxin according to claim 10, wherein the cytotoxic moiety of said immunotoxin is the A-chain of ricin.

12. An immunotoxin according to claim 10, wherein the cytotoxic moiety of said immunotoxin is the A-chain of abrin.

13. An immunotoxin according to claim 10, wherein the cytotoxic moiety of said immunotoxin is the A-chain of the diphteria toxin.

14. A method for specific detection of HIV-I infected cells in human blood or tissue culture fluid, characterized in that the antibody according to claim 1 is used.

## Patentansprüche

1. Antikörper 3D6, der sich selektiv an ein Hüllprotein von HIV-I bindet und durch Hybridoma mit der ECACC-Zugriffs-Nr. 87110301 erzeugt ist.

2. Antikörper nach Anspruch 1, die sich auch selektiv an mit HIV-I (HTLV III/LAV) infizierte Zellen binden.

3. Antikörper nach Anspruch 1, der gegen mit HIV-I infizierte H-9-Zellen eine TCID 50% von weniger als 10 nM zeigt, wenn er an eine Rizin-A-Kette konjugiert ist.

4. Antikörper nach Anspruch 1 oder 2, der ein G-Isotop aufweist.

5. Maus x menschliches Hybridoma, das einen monoklonalen menschlichen Antikörper nach einem der Ansprüche 1 bis 4 produziert, sowie Abkömmling besagter Hybridomas.

6. Immunotoxin mit einem Konjugat des Antikörpers nach einem der Ansprüche 1 bis 4 und einer zytotoxischen Gruppe.

7. Immunotoxin nach Anspruch 6, bei dem die zytotoxische Gruppe des Immunotoxins die A-Kette von Rizin ist.

8. Immunotoxin nach Anspruch 6, bei dem die zytotoxische Gruppe des Immunotoxins die A-Kette von Abrin ist.

9. Immunotoxin nach Anspruch 6, bei dem die zytotoxische Gruppe des Immunotoxins die A-Kette des Diphterie-Toxins ist.

10. Immunotoxin mit einem Konjugat des Antikörpers nach einem der Ansprüche 1 bis 4 und einer zytotoxischen Gruppe.

11. Immunotoxin nach Anspruch 10, bei dem die zytotoxische Gruppe des Immunotoxins die A-Kette von Rizin ist.

12. Immunotoxin nach Anspruch 10, bei dem die zytotoxische Gruppe des Immunotoxins die A-Kette von Abrin ist.

13. Immunotoxin nach Anspruch 10, bei dem die zytotoxische Gruppe des Immunotoxins die A-Kette des Diphterie-Toxins ist.

14. Verfahren zur spezifischen Feststellung von mit HIV-I infizierten Zellen in menschlichem Blut oder dem Fluid einer Gewebekultur, dadurch gekennzeichnet, daß ein Antikörper nach Anspruch 1 verwendet wird.

## Revendications

1. Anticorps 3D6 qui se lie sélectivement à une protéine d'enveloppe du HIV-I produit par un hybridome ayant le numéro d'entrée à l'ECACC 87110301.

2. Anticorps selon la revendication 1, qui se lie également sélectivement aux cellules infectées par le HIV-I (HTLV III/LAV).

3. Anticorps selon la revendication 1, qui présente une dose immunisant les cultures de tissus (TCID) à 50 % contre les cellules H-9 infectées par le HIV-I de moins de 10 nM, lorsqu'il est conjugué à la chaîne A de la ricine.

4. Anticorps selon les revendications 1 ou 2, qui a un isotype G.

5. Hybridome de souris x homme, qui produit un anticorps monoclonal humain selon l'une des revendications 1 à 4 et la descendance de ces hybridomes.

6. Immunotoxine comprenant un conjugué de l'anticorps selon l'une des revendications 1 à 4 et une partie cytotoxique.

7. Immunotoxine selon la revendication 6, dans laquelle la partie cytotoxique de cette immunotoxine est la chaîne A de la ricine.

8. Immunotoxine selon la revendication 6, dans laquelle la partie cytotoxique de cette immunotoxine est la chaîne A de l'abrine.

9. Immunotoxine selon la revendication 6, dans laquelle la partie cytotoxique de cette immunotoxine est la chaîne A de la toxine diphtérique.

10. Immunotoxine comprenant un conjugué de l'anticorps selon l'une des revendications 1 à 4 et une partie cytotoxique.

11. Immunotoxine selon la revendication 10, dans laquelle la partie cytotoxique de cette immunotoxine est la chaîne A de la ricine.

12. Immunotoxine selon la revendication 10, dans laquelle la partie cytotoxique de cette immunotoxine est la chaîne A de l'abrine.

13. Immunotoxine selon la revendication 10, dans laquelle la partie cytotoxique de cette immunotoxine est la chaîne A de la toxine diphtérique.

14. Méthode de détection spécifique des cellules infectées par le HIV-I dans le sang humain ou le fluide des cultures de tissus, caractérisée en ce que l'on utilise l'anticorps selon la revendication 6.
